# EUROPÄISCHE PATENTSCHRIFT

(11) **EP 0 597 358 B1**
(45) Veröffentlichungstag und Bekanntmachung des Hinweises auf die Patenterteilung: **09.02.2000**
(21) Anmeldenummer: 93117742.2
(22) Anmeldetag: 02.11.1993
(51) Int. Cl.: C07H 15/04, C07H 1/08

(54) **Verfahren zur quantitativen Aufreinigung von Glycolipiden**
Method for the quantitative purification of glycolipids
Procédé de purification quantitative de glycolipides

(30) Priorität: 05.11.1992 DE 4237334
(43) Veröffentlichungstag der Anmeldung: 18.05.1994
(73) Patentinhaber: Aventis Research & Technologies GmbH & Co. KG, 65926 Frankfurt am Main (DE)
(72) Erfinder: Mixich, Johann, Dr., D-65779 Kelkheim/Taunus (DE); Rothert, Reinhardt, D-65527 Niedernhausen (DE); Wullbrandt, Dieter, Dr., D-65719 Hofheim/Taunus (DE)

(56) Entgegenhaltungen:
- EP-A- 0 282 942
- FR-A- 2 110 410

## Beschreibung

Glycolipide sind aus einem Fettsäure- und einem Zuckerrest zusammengesetzt. Eines der bekanntesten Glycolipide ist das Trehaloselipid (Suzuki et al., 1969, Agric. Biol. Chem., 33, 1619-1627). Es besteht aus dem Disaccharid Trehalose und zwei β-Hydroxy-α-verzweigten Fettsäuren (Crynomycolsäure), die mit Hydroxy-Gruppen des Zuckers verestert sind. Glycolipide, die Rhamnose und β-Hydroxyfettsäuren enthalten, werden in EP 0 153 634 beschrieben. Das erste Rhamnolipid, dessen Struktur bestimmt wurde, besteht aus zwei Molekülen L-Rhamnose und zwei Molekülen β-Hydroxydecansäure (Ewards und Hayashi, 1965, Arch. Biochem. Biophys., 111, 415-421).

Die bisher bekannten Glycolipide haben je nach Zucker- und Fettsäureanteil ein Molekulargewicht zwischen 250 und 2000 Dalton. Sie werden beispielsweise von Bakterien ins umgebende Nährmedium abgegeben und können als Mischung von mehreren verschiedenen Glycolipiden angehäuft werden (EP 0 153 634).

Glycolipide können beispielsweise als Emulgatoren, Biotenside oder Stabilisatoren für Emulsionen verwendet werden.

Die Reinigungsverfahren von Glycolipiden beruhen auf Extraktions-, Kristallisations-und Chromatographieprozessen (EP 0 282 942; US 4 812 272; FR 2110710). Bei diesen Verfahren fallen jedoch große Mengen Lösemittel an. Mulligan und Gibbs (J. Chem. Tech. Biotechnol., 1990, 47, 23-29) beschreiben ein Ultrafiltrationsverfahren zur Reinigung von Biotensiden, wie Surfactin und Rhamnolipiden. Das dort beschriebene Rückhaltevermögen der Membranen nimmt jedoch mit zunehmender Porengröße ab. Dies führt bei Membranen mit einer Trenngrenze von 30 000 Dalton zu Verlusten an Glycolipiden von mehr als 77 %.

Ein weiteres Membrantrennverfahren, bei dem diese Verluste nicht auftreten, wird in der Anmeldung WO 9205183 beschrieben. Dieses Verfahren besitzt den Nachteil, daß mit zunehmender Aufreinigung und Aufkonzentrierung die Permeatleistung abnimmt.

Die Aufreinigung von Rhamnolipiden kann auch aus der Kulturlösung erfolgen, indem die Kulturlösung angesäuert und anschließend der gesamte Ansatz 2 bis 3 Tage auf etwa 4°C gekühlt wird (Jarvis, F.G., Johnson, M.J., J. Amer. Chem. Soc. 71, 4124 (1949)).

Auch Davis et al. (US Patent Nr. 4,933,281) reinigen Rhamnolipid aus der Kulturlösung durch Ansäuern der Kulturlösung und nachfolgender Kühlung des gesamten Ansatzes. In Beispiel 3 wird beschrieben, daß die Kulturlösung mit Schwefelsäure auf pH 2,5 eingestellt und über Nacht bei 4°C gehalten wird. Die Ausbeute an Rhamnolipid nach Zentrifugation beträgt 73 %.

Überraschend wurde nun gefunden, daß Glycolipide quantitativ aufgereinigt werden können, indem man die glycolipidenthaltende Lösung ansäuert, den gesamten Ansatz erhitzt und nach dem anschließenden Abkühlen des Ansatzes diesen zentrifugiert.

Die Erfindung betrifft somit ein Verfahren zur quantitativen Aufreinigung von Glycolipiden, das dadurch gekennzeichnet ist, daß die Aufreinigung der Glycolipide durch Ansäuern der glycolipidenthaltenden Lösung auf pH ≤ 5,0, anschließendem Erhitzen des Ansatzes auf 60°C - 130°C, nachfolgendem Abkühlen des Ansatzes auf eine Temperatur ≤ 50°C und Zentrifugation des Ansatzes zur Abtrennung der glycolipidenthaltenen Phase, erfolgt.

Die Erfindung wird im folgenden detailliert beschrieben, insbesondere in ihren bevorzugten Ausführungsformen.

"Quantitativ" bedeutet, daß die Glycolipidausbeute zwischen 90 % und 99 % liegt, bezogen auf die in der Ausgangslösung enthaltene Glycolipidmenge.

Die Definition für die Abkürzung "g" lautet wie folgt:
g = 9,80665 m/s². Dieser Wert wurde im jahr 1901 von der 3. Konferenz für Maß und Gewicht als festgelegter Normwert der Fallbechleunigung definiert.

Das erfindungsgemäße Verfahren kann für Aufreinigungen im Labormaßstab (Milliliter- bis Literbereich) und für den industriellen Maßstab (Kubikmetermaßstab) eingesetzt werden.

Glycolipide können in Pflanzen oder Bakterien vorkommen. Für die Herstellung der Glycolipide kommt bevorzugt die Fermentation von Mikroorganismen in Betracht. Dazu werden die Mikroorganismen in an sich bekannter Weise kultiviert. Die in das Kulturmedium ausgeschiedenen Glycolipide werden nach beendeter Fermentation mit Hilfe des erfindungsgemäßen Verfahrens quantitativ aufgereinigt.

Das erfindungsgemäße Verfahren wird vorzugsweise für die Reinigung von Rhamnolipiden eingesetzt. Die Reinigung von α-L-Rhamnopyranosyl-β-hydroxydecansäure,
2-O-α-L-Rhamnopyranosyl-α-L-rhamnopyranosyl-β-hydroxydecansäure,
2-O-α-L-Rhamnopyranosyl-α-L-rhamnopyranosyl-β-hydroxydecanoyl-β-hydroxydecansäure oder
α-L-Rhamnopyranosyl-β-hydroxydecanoyl-β-hydroxydecansäure, ist besonders bevorzugt.

Das erfindungsgemäße Verfahren besteht aus den aufeinanderfolgenden Schritten:
1. Die glycolipidenthaltende Lösung, vorzugsweise die durch die Fermentation von Bakterien entstehende Kulturlösung, wird auf pH ≤ 5,0 angesäuert. Vorzugsweise wird die Lösung auf pH 2,5 - 4,0 eingestellt.
   Das Ansäuern kann mit Hilfe aller, dem Chemiker bekannten Säuren erfolgen (z.B. H₂SO₄, Oleum, HCl, H₃PO₄ usw.).
   Für das erfindungsgemäße Verfahren kann Säure in beliebiger Konzentration verwendet werden. Ist eine Verdünnung der glycolipidenthaltenden Lösung unerwünscht, muß konsequenterweise stark konzentrierte Säure eingesetzt werden.
   Die Zugabe der Säure erfolgt unter Rühren der Lösung und permanenter Kontrolle des pH-Wertes.
2. Nach dem Ansäuern wird der Ansatz auf 60°C - 130°C, vorzugsweise 90°C - 110°C, erhitzt. Die für den Erhitzungsprozeß benötigte Zeitdauer ist variabel. Beispielsweise kann der Erhitzungsprozeß sofort nach dem Erreichen der gewünschten Maximaltemperatur gestoppt werden.
   Sind in dem Ansatz pathogene Keime enthalten, wird die zur Inaktivierung der Keime notwendige Temperatur bis zur Inaktivierung dieser gehalten. Dem Fachmann ist bekannt, welche Keime pathogen sind und welche Temperatur und Zeitdauer zur Inaktivierung notwendig ist.
3. Im Anschluß an den Ansäuerungs- und Temperierungsschritt erfolgt das Kühlen des gesamten Ansatzes auf ≤ 50°C, vorzugsweise 20°C - 30°C.
   Die Dauer des Kühlvorgangs wird bestimmt durch die Art der Kühlung (z.B. im Kühlschrank bei kleineren Ansätzen, Kühlen von Großfermentern über Wärmetauscher) und/oder dem verwendeten Kühlmittel (Flußwasser, Kühlsole).
   Zweckmäßigerweise wird der Kühlvorgang so durchgeführt, daß ein wirtschaftliches Verhältnis zwischen den Kosten für die einzusetzende Energie und der Zeitdauer des Kühlvorgangs vorhanden ist.
4. Im letzten Schritt wird der auf eine Temperatur von ≤ 50°C, vorzugsweise 20 - 30°C, gekühlte Ansatz zentrifugiert. Die Zentrifugation erfolgt ≥ 500 g bis zur Phasentrennung.
   Die Glycolipide befinden sich nach der Zentrifugation in der Unteren Phase.

Wird das Verfahren im Labormaßstab durchgeführt, erhält man die untere Phase zur weiteren Verarbeitung durch einfaches Abgießen der oberen Phase.

Bei der Durchführung des Verfahrens im industriellen Maßstab werden handelsübliche Separatoren (z.B. von Fa. Westfalia oder Fa. Alpha-Laval, Deutschland) und Decanter zur Trennung der Phasen und damit zur Gewinnung der glycolipidenthaltenden Phase eingesetzt.

Die Glycolipidausbeute liegt zwischen 90 % - 99 %, bezogen auf die in der Ausgangslösung enthaltene Glycolipidmenge.

Als Ausgangslösung wird die Lösung bezeichnet, die für Schritt 1 des erfindungsgemäßen Verfahrens eingesetzt wird.

Vergleichsversuche haben gezeigt, daß keine quantitative Aufreinigung von Glycolipiden erreicht wird, wenn die glycolipidenthaltende Lösung zuerst auf 60°C - 130°C erhitzt und nach dem Abkühlen auf Raumtemperatur abschließend auf pH ≤ 5,0 angesäuert wird.

Das erfindungsgemäße Verfahren dient, wie oben schon ausgeführt, zur quantitativen Aufreinigung von Glycolipiden. Selbstverständlich kann es auch zur Konzentrierung eingesetzt werden.

Das erfindungsgemäße Verfahren besitzt gegenüber denen im Stand der Technik beschriebenen Verfahren Vorteile:
- Das erfindungsgemäße Verfahren ist zeitsparend, da der im Stand der Technik (US Patent Nr. 4,933,281) beschriebene Verfahrensschritt des Abkühlens auf 4°C über Nacht, also für 12 - 16 Stunden, wegfällt.
- Die Ausbeute wird von 73 %, wie im US-Patent Nr. 4,933,281 beschrieben, auf 90 - 99 % gesteigert.
- Bei Verwendung von pathogenen Keimen in der Fermentation erfolgt die in vielen Ländern gesetzlich vorgeschriebene Keimabtötung wahrend des Erhitzungsprozesses des erfindungsgemäßen Verfahrens, welches demzufolge einen andernfalls anfallenden, zusätzlichen Arbeitsschritt einspart.

### Beispiele

### Beispiel 1

### Batch-Fermentation im Industriemaßstab zur Gewinnung von L-Rhamnose

a) Vorkultur
Eine erste Vorkultur des Stammes Pseudomonas aeruginosa DSM 7107 in 4 l Vorkultur-Nährlösung (Tab. 1) wird in Schüttelkolben hergestellt (2 l-Erlenmeyerkolben mit je 500 ml Nährlösung, 30°C, 200 UpM, 20 h). Die gesamte 1. Vorkultur wird zum Beimpfen der 2. Vorkultur (350 l) verwendet.
Dazu wird in einem 450 l-Fermenter mit 350 l komplexer Vorkultur-Nährlösung (Tabelle 1) der Stamm DSM 7107 aerob bei einer Belüftungsrate von 180 l Luft/min einer Rührergeschwindigkeit von 300 UpM, bei einer Temperatur von 28 °C 16 Stunden lang fermentiert.

**Tab. 1**

| Vorkultur - Nährlösung: |
|---|
| 10 g/l Glucose |
| 5 g/l Caseinpepton |
| 1 g/l Hefeextrakt |
| 0,5 g/l NaCl. |

Die gesamte 2. Vorkultur wird zur Beimpfung der Hauptkultur verwendet.
b) Hauptkultur
In einem Fermenter mit ca. 30m³ Nennvolumen werden 17 m³ der in Tabelle 2 angegebenen Nährlösung zubereitet:

**Tab. 2**

| Hauptkultur - Nährlösung: |
|---|
| 6,47 g/l 75%ige H₃PO₄ |
| ca. 8,94 g/l 33%ige NaOH |
| 0,5 g/l MgSO₄·7H₂O |
| 1 g/l KCl |
| 15 g/l NaOH |
| 125 g/l Sojaöl |

Dazu wird nach Vorlage der benötigten Wassermenge mit H₃PO₄ und NaOH ein pH-Wert von 6,8 eingestellt. Nach Zugabe der restlichen Nährlösungsbestandteile wird mit H₂SO₄ der pH-Wert auf pH 6,2 korrigiert.
Nach 45 minütiger Sterilisation hat sich ein pH-Wert von ca. 6,3 eingestellt. In einem separaten Behälter wird eine Lösung mit Spurenelementen (Tab. 3) sterilisiert. Dazu werden in 150 l deionisiertem Wasser folgende Substanzen gelöst und sterilisiert:

**Tab. 3**

| Spurenelementelösung: |
|---|
| 2 mg/l Natriumcitrat·2H₂O |
| 0,28 mg/l FeCl₃·6H₂O |
| 1,4 mg/l ZnSO₄·7H₂O |
| 1,2 mg/l CoCl₂·6H₂O |
| 1,2 mg/l CuSO₄·5H₂O |
| 0,8 mg/l MnSO₄·1H₂O |
| deionisiertes Wasser |
| Konzentrationsangaben beziehen sich auf 1 l Hauptkultur |

Diese Spurenelemente-Lösung wird vor der Beimpfung und 3 weitere Male nach ca. 20, 40 und 70 Stunden Fermentationsdauer dem Hauptfermenter unter sterilen Bedingungen zugegeben.
Als Inokulum wird der gesamte Inhalt des Vorfermenters (350 l) verwendet. Die Fermentationstemperatur beträgt 30°C. In den ersten 10 Fermentationsstunden wird mit 250 m³ Luft/Stunde belüftet, von der 10. bis zur 30. Stunde mit 400 m³/h und ab der 30. Stunde mit 100 - 75 m³/h.
Zur Schaumbekämpfung wird ein separat sterilisiertes Silikonantischaummittel VP 1133 (Fa. Wacker) verwendet, das in Abhängigkeit vom Schäumungsverhalten der Fermentationslösung unter Zuhilfenahme einer Schaumelektrode in den Fermenter portionsweise dosiert wird.
Als Rührorgan wird ein Radialrührer mit vier Turbinen, die einen Durchmesser von 1040 mm aufweisen eingesetzt (Rührer ⌀: Fermentor ⌀ = 0,4 : 1). Die Drehzahl beträgt in den ersten 10 Fermentationsstunden 50 UpM, ab der 10. Stunde 75 UpM.
Unter den oben genannten Fermentationsbedingungen lassen sich in 167 Std. Fermentationsdauer pro Liter Kulturlösung ca. 78 g Rhamnolipide und ein Rhamnosegehalt von ca. 32 g Rhamnose erzeugen.

### Beispiel 2:

### Fed-Batch-Fermentation im Industriemaßstab zur Gewinnung von L-Rhamnose

18,5 m³ Hauptkulturmedium mit der Zusammensetzung aus Beispiel 1 werden mit 350 l Vorkultur (ebenfalls in Beispiel 1 beschrieben) beimpft. Als Produktionsstamm wird der Stamm Pseudomonas aeruginosa DSM 7108 eingesetzt. Vor der Beimpfung und nach 20, 40, 70 und 120 Stunden Fermentationsdauer wird unter sterilen Bedingungen je eine Spurenelementelösung (siehe Beispiel 1) zugegeben.

Die Belüftungsraten werden folgendermaßen variiert: Bei Fermentationsstart 250 m³/h, nach 10 Fermentationsstunden 350 m³/h und nach 30 Fermentationsstunden in Abhängigkeit von der Intensität der Schaumbildung 100-130 m³/h Luft.
In den ersten 10 Fermentationsstunden wird mit 50 UpM gerührt, danach mit 75 UpM. Von der 72sten bis zur 109ten Fermentationsstunde werden kontinuierlich weitere 564 l Sojaöl zudosiert.
Die Schaumbekämpfung erfolgt in gleicher Weise wie in Beispiel 1 beschrieben wurde. Unter den genannten Fermentationsbedingungen werden in 9 Tagen in der Kulturlösung 95 g/l Rhamnolipide und ein Gehalt an Rhamnose von 39 - 40 g/l erzeugt.

### Beispiel 3

50 g der aus den Beispielen 1 oder 2 gewonnenen Fermenterlösung wird mit 6 N H₂SO₄ auf pH 3,0 angesäuert, auf 100°C erhitzt und 60 Minuten bei dieser Temperatur gehalten. Anschließend wird die Lösung auf 20°C abgekühlt und in einer Laborzentrifuge für 15 Minuten bei 3200 g zentrifugiert.
Die Zentrifugation führt hier zur Bildung von zwei, voneinander scharf getrennten Phasen. Die untere Phase mit einem Sedimentvolumen von 24 %, die man durch Dekantieren der oberen Phase erhält, enthält über 98 % des in der Fermentationsprobe enthaltenen Rhamnolipidgemisches.

### Beispiel 4

Dieses Beispiel wird analog zu Beispiel 3 durchgeführt, nur mit dem Unterschied, daß das Ansäuern nach dem Erhitzen erfolgt.

50 g der aus Beispiel 1 gewonnenen Fermenterlösung wird auf 100°C erhitzt und 60 Minuten bei dieser Temperatur gehalten. Dann wird die Lösung auf 20°C abgekühlt und mit 6 N H₂SO₄ auf pH 3,0 eingestellt. Durch Zentrifugation in einer Laborzentrifuge (15 Minuten bei 3200 g) kommt es nicht zur Ausbildung von zwei Phasen, demzufolge kann das Rhamnolipidgemisch nicht abgetrennt werden.

## Patentansprüche

1. Verfahren zur quantitativen Aufreinigung von Glycolipiden, dadurch gekennzeichnet, daß die Aufreinigung der Glycolipide durch
- Ansäuern der glycolipidenthaltenden Lösung auf pH ≤ 5,0,
- anschließendem Erhitzen des Ansatzes auf 60°C - 130°C,
- nachfolgendem Abkühlen des Ansatzes auf ≤ 50°C und
- Zentrifugation des Ansatzes zur Sedimentation der glycolipidenthaltenden Phase,
erfolgt.

2. Verfahren nach Anspruch 1, dadurch gekennzeichnet, daß Rhamnolipide aufgereinigt werden.

3. Verfahren nach Anspruch 2, dadurch gekennzeichnet, daß Rhamnolipide aus einer bakteriellen Kulturlösung aufgereinigt werden.

4. Verfahren nach Anspruch 2, dadurch gekennzeichnet, daß α-L-Rhamnopyranosyl-β-hydroxydecansäure, 2-O-α-L-Rhamnopyranosyl-α-L-rhamnopyranosyl-β-hydroxydecansäure, 2-O-α-L-Rhamnopyranosyl-α-L-rhamnopyranosyl-β-hydroxydecanoyl-β-hydroxydecansäure oder α-L-Rhamnopyranosyl-β-hydroxydecanoyl-β-hydroxydecansäure aufgereinigt werden.

5. Verfahren nach einem oder mehreren der Ansprüche 1 bis 4, dadurch gekennzeichnet, daß die glycolipidenthaltende Lösung auf pH 2,5 - 4,0 angesäuert wird.

6. Verfahren nach einem oder mehreren der Ansprüche 1 bis 4, dadurch gekennzeichnet, daß der Ansatz auf 90°C - 110°C erhitzt wird.

7. Verfahren nach Anspruch 1 bis 4, dadurch gekennzeichnet, daß das Abkühlen des Ansatzes auf 20°C bis 30°C erfolgt.

8. Verfahren nach Anspruch 1 bis 4, dadurch gekennzeichnet, daß die Zentrifugation des Ansatzes ≥ 500 g bis zur Phasentrennung erfolgt.

## Claims

1. A process for quantitatively purifying glycolipids wherein the purification of the glycolipids is effected by
- acidifying the glycolipid-containing solution to pH ≤ 5.0,
- subsequently heating the batch to 60°C - 130°C,
- then cooling the batch down to ≤ 50°C and
- centrifuging the batch to sediment the glycolipid-containing phase.

2. The process as claimed in claim 1, wherein rhamnolipids are purified.

3. The process as claimed in claim 2, wherein rhamnolipids are purified from a bacterial culture solution.

4. The process as claimed in claim 2, wherein α-L-rhamnopyranosyl-β-hydroxydecanoic acid, 2-O-α-L-rhamnopyranosyl-α-L-rhamnopyranosyl-β-hydroxydecanoic acid, 2-O-α-L-rhamnopyranosyl-α-L-rhamnopyranosyl-β-hydroxydecanoyl-β-hydroxydecanoic acid or α-L-rhamnopyranosyl-β-hydroxydecanoyl-β-hydroxydecanoic acid are purified.

5. The process as claimed in one or more of claims 1 to 4, wherein the glycolipid-containing solution is acidified to pH 2.5 - 4.0.

6. The process as claimed in one or more of claims 1 to 4, wherein the batch is heated to 90°C - 110°C.

7. The process as claimed in claims 1 to 4, wherein the batch is cooled down to 20°C to 30°C.

8. The process as claimed in claims 1 to 4, wherein the batch is centrifuged at ≥ 500 g until the phases are separated.

## Revendications

1. Procédé de purification quantitative de glycolipides, est caractérisé en ce que la purification des glycolipides est effectuée en suivant les étapes consistant
- à acidifier la solution contenant des glycolipides à pH ≤ 5,0,
- à chauffer ensuite le lot à 60°C - 130°C,
- à refroidir ensuite le lot à une température ≤ 50°C et
- à centrifuger le lot pour sédimenter la phase contenant les glycolipides.

2. Procédé selon la revendication 1, caractérisé en ce que les rhamnolipides sont purifiés.

3. Procédé selon la revendication 2, caractérisé en ce que les rhamnolipides sont purifiés à partir d'une solution de culture bactérienne.

4. Procédé selon la revendication 2, caractérisé en ce que l'acide α-L-rhamnopyranosyl-β-hydroxydécanoïque, l'acide 2-O-α-L-rhamnopyranosyl-α-L-rhamnopyranosyl-β-hydroxydécanoïque, l'acide 2-O-α-L-rhamnopyranosyl-α-L-rhamnopyranosyl-β-hydroxydécanoyl-β-hydroxydécanoïque ou l'acide α-L-rhamnopyranosyl-β-hydroxydécanoyl-β-hydroxydécanoïque est purifié.

5. Procédé selon une ou plusieurs des revendications 1 à 4, caractérisé en ce que la solution contenant les glycolipides est acidifiée à pH 2,5 - 4,0.

6. Procédé selon une ou plusieurs des revendications 1 à 4, caractérisé en ce que le lot est chauffé à 90°C - 110°C.

7. Procédé selon la revendication 1 à 4, caractérisé en ce qu'on effectue le refroidissement du lot à 20°C à 30°C.

8. Procédé selon la revendication 1 à 4, caractérisé en ce qu'on réalise la centrifugation du lot à ≥ 500 g jusqu'à la séparation des phases.
